# EUROPÄISCHE PATENTSCHRIFT

(11) **EP 1 361 894 B1**
(45) Veröffentlichungstag und Bekanntmachung des Hinweises auf die Patenterteilung: **26.04.2006**
(21) Anmeldenummer: 02717950.6
(22) Anmeldetag: 15.02.2002
(51) Int. Cl.: A61K 45/00, A61P 11/00, A61P 9/00

(54) **VERWENDUNG EINES POLYFUNKTIONELLEN WIRKSTOFFGEMISCHES ALS TABAKRAUCHSCHADSTOFFANTAGONIST**
USE OF A POLYFUNCTIONAL ACTIVE SUBSTANCE MIXTURE AS AN ANTAGONIST AGAINST HARMFUL SUBSTANCES CONTAINED IN TOBACCO SMOKE ACTING AS A HEALTH PROTECTING AGENT DURING SMOKING
UTILISATION D'UN MELANGE POLYFONCTIONNEL DE SUBSTANCES ACTIVES AYANT UNE FONCTION D'ANTAGONISTE DE SUBSTANCES NOCIVES CONTENUES DANS LA FUMEE DE TABAC ET SERVANT D'AGENT DE PROTECTION DE LA SANTE DANS LE CADRE D'UNE ABSORPTION DE FUMEE DE TABAC

(30) Priorität: 16.02.2001 DE 10107731
(43) Veröffentlichungstag der Anmeldung: 19.11.2003
(73) Patentinhaber: Hecht, Karl, 12527 Berlin (DE); Tech, Egon, 18334 Bad Sülze (DE)
(72) Erfinder: HECHT, Karl, 12527 Berlin (DE); TECH, Egon, 18334 Bad Sülze (DE); DEHMLOW, Ronald, 16547 Birkenwerder (DE); ARBEIT, Ekkard, 13059 Berlin (DE)
(74) Vertreter: Baumbach, Friedrich
(86) Internationale Anmeldenummer: PCT/DE2002/000563
(87) Internationale Veröffentlichungsnummer: WO 2002/066043

(56) Entgegenhaltungen:
- EP-A- 0 577 143
- WO-A-96/00019
- DE-A- 19 632 521
- DE-C- 19 512 227
- US-A- 5 330 972

## Beschreibung

Die Erfindung betrifft die Verwendung eines polyfunktionellen Wirkstoffgemisches mit antiinflammativer, spasmolytischer und Antistresswirkung, welches aus einer Fraktion spezifischer Peptide mit Molgewichten bis 10.000 Dalton sowie einer Fraktion mit essentiellen und nichtessentiellen Aminosäuren besteht und aus einem multifaktoriellen Abwehr-Modulatorgemisch gewonnen wurde, als Tabakrauchschadstoffantagonist mit gesundheitsschützender Funktion beim Tabakrauchen.

Es wird bevorzugt als Inhalat (Adhäsionsprinzip, Vernebelung) appliziert und führt zur Abschwächung, Beseitigung und Verhinderung von tabakrauchschadstoffinduzierten Entzündungen der Schleimhäute, von Regulationsstörungen der Organsysteme mit glatter Muskulatur (Blutgefäße, Bronchien, Ösophagus, Blase, Magen, Därme u.a.), der Funktionen des Herzmuskels, des zentralen und peripheren Nervensystems.
Das polyfunktionelle Wirkstoffgemisch wird aus einem multifaktoriellen Abwehr - Modulatorengemisch gewonnen.

Zur Tabakschadstoffwirkung auf die Gesundheit und zur pathogenen Wirkung des Tabakrauchens liegen unzählige wissenschaftliche Arbeiten vor, so dass sich in letzter Zeit Regierungen und Gerichte mit dieser Problematik auseinandersetzen mußten und noch immer müssen. Hierfür sollen lediglich einige Beispiele angeführt werden.
1. EU-Kommissar für Gesundheit und Verbraucherschutz David Byrne (Focus 11/2000 S. 362/363)
   "Am Tabakkonsum sterben jährlich eine halbe Million EU-Bürger - das ist ein Toter pro Minute."
2. In der Weltgesundheitsorganisation wird über eine Antitabak-Konvention verhandelt (Quelle dieselbe).
3. Mütterliches Rauchen erhöht pränatale Belastungen mit karzinogenen und teratogenen Stoffen. (Deutsches Ärzteblatt 97/39 2000 S. C 1873)
4. Ein Geschworenengericht in den USA verurteilte die amerikanische Tabakindustrie zur Zahlung von 145 Milliarden Dollar an erkrankte Raucher. Das ist die höchste in den USA jemals verhängte Zivilstrafe (Focus 51/2000 S. 168)

### Kurze Zusammenfassung der wesentlichen pathogenen Folgen des Tabakrauchens

1. Es ist bekannt, dass Tabakrauchen, vor allem Zigarettenrauchen Schadstoffwirkungen haben, die chronische Entzündungen der Schleimhäute des Verdauungs- und Atmungssystems und auch der Augen zur Folge haben. Vor allem sind die Schleimhäute des Mundes, der Nase, des Rachens und Kehlkopfes, der Bronchien und der Bronchiolen, der Speiseröhre, des Magens, des Zwölffingerdarms, des Dünndarmes und auch des Dickdarmes betroffen. Nicht wenige Menschen leiden an einer chronischen Konjunktivitis infolge Zigarettenrauchens. Diese tabakrauchschadstoffinduzierten Entzündungen haben häufig Geschwürbildungen im Verdauungstrakt, die Verhinderung bzw. Einschränkung der Aufnahme von Vitamin C und Betacarotin - Vitamin A - (woraus sich Vitaminmangelerkrankungen ergeben), Infektionskrankheiten der Atmungsorgane u.a. zur Folge, woraus ein hoher Anfall von Krankheitskosten entsteht (siehe Gerichtsprozesse in den USA).
2. Es ist bekannt, dass Schadstoffe des Tabakrauchens (es sind ca. 200 nachgewiesen) Entzündungen der Schleimhäute, Störungen der glatten Muskulatur in Form von Kontraktion oder Spasmen der Blutgefäße, der Bronchien und des Verdauungssystems nach sich ziehen. Mangeldurchblutung des Herzens (Herzmuskelinfarkt) der Haut, des Gehirns, der Extremitäten (Raucherbein u.a.) und vor allem pectangiose Beschwerden der Rauchenden sind die häufigsten Störungen, wie einschlägige Statistiken ausweisen.
3. Es ist bekannt, dass Tabakrauchstoffe auch als Streßfaktoren wirken und z.B. das Nebennierenmarkhormon Adrenalin verstärkt in die Blutbahn bringen, wodurch generalisiert Disstress ausgelöst wird. Hierbei werden einerseits die unter 1.u.2. ausgeführten pathologischen Erscheinungen stimuliert und somit verstärkt und andererseits die vom Disstress bekannten Regulationsstörungen z.B. des Immunsystems und Transmittersystems (einschließlich Regulations- und Neuropeptide) hervorgerufen bzw. verursacht.
4. Heute ist bekannt, dass Rauchen eine Sucht darstellt und das Abgewöhnen gewöhnlich nicht nur sehr schwer fällt, sondern von vielen schweren pathologischen Entzugserscheinungen begleitet wird. Deshalb rauchen viele Raucher weiter, obgleich Ihnen das Gesundheitsrisiko bekannt ist.
5. Aus diesen wissenschaftlichen Erkenntnissen resultiert die logische Schlußfolgerung, dass Rauchen die Gesundheit schädigt und dass jede Zigarettenschachtel oder Werbung die Warnung vor dieser Schädigung erfolgen muß.
6. Der Tabakraucher ist süchtig und beabsichtigt aus diesen Gründen weiter zu rauchen, obgleich er weiß, dass er seine Gesundheit schädigt, so befindet er sich in einem, häufig auch unbewußt ablaufenden permanentem Konflikt.
7. Ein Mittel, welches vor gesundheitlichen Schäden beim Tabakrauchen schützt, gibt es bisher nicht, obgleich es unbedingt angezeigt ist und von großem Nutzen wäre, um den schon süchtigen Raucher, erstens den belastenden Konflikt zu nehmen und zweitens vor weiteren gesundheitlichen Schäden zu bewahren.

Die Aufgabe der Erfindung bestand deshalb darin, einen Tabakrauchschadstoffantagonisten mit gesundheitsschützender Funktion zu finden, der selbst nicht Gesundheits- und Sucht- gefährdend ist, den Geschmack der Rauchware und den Rauchgenuß nicht beeinträchtigt und unkompliziert einsetzbar ist und zur Abschwächung, Beseitigung und Verhinderung Tabakrauchschadstoff-induzierter
- Entzündungen der Schleimhäute
- Regulationsstörungen
- der Funktion der Organsysteme mit glatter Muskulatur (Bronchien, Blutgefäße, Ösophagus, Blase, Magen, Därme u.a.)
- der Funktionen der Herztätigkeiten sowie
- der Funktionen des zentralen und peripheren Nervensystems dient.

Diese Aufgabe konnte durch ein polyfunktionelles Wirkstoffgemisch gelöst werden, das sich durch antiinflammative, spasmolytische und Antistresswirkung auszeichnet und welches aus einer Fraktion spezifischer Peptide mit Molgewichten bis 10.000 Dalton und/oder einer Fraktion mit essentiellen und nichtessentiellen Aminosäuren besteht, die aus einem multifaktoriellen Abwehr- Modulatorgemisch gewonnen wurde.

Das polyfunktionelle Wirkstoffgemisch wird zur Anwendung bevorzugt in die Rauchware eingefügt und als Inhalat (Adhäsionsprinzip, Vernebelung via Atemwegen und Atemorgan) über ein Mundstückfilter der Rauchware (Zigarette, Zigarre, Zigarillo, Tabakpfeife) dem Körper zugeführt.

Multifaktorielle Abwehr-Modulatorengemische mit antiinflammativer Wirkung sind an sich bekannt. Verwendung findet ein im wesentlichen in DE 195 12 227 C1 beschriebenes Modulatorengemisch, das sich jedoch von dem Gemisch aus DE 195 12 227 C1 dadurch unterscheidet , das es eine Fraktion spezifischer Peptide mit einem Molekulargewicht bis 10.000 Dalton und/oder einer Fraktion mit essentiellen und nichtessentiellen Aminosäuren darstellt. Seine Herstellung erfolgt analog DE 195 12 227 C1, d.h. es wird aus somatischen Zellen gewonnen, wobei im Unterschied zu DE 195 12 227 C1 die Fraktion spezifischer Peptide mit einem Molekulargewicht bis 10.000 Dalton und/oder mit essentiellen und nichtessentiellen Aminosäuren durch Ultrazentrifugation bei einer Laufzeit von 24 Stunden isoliert wird. Das heißt, das polyfunktionelle Wirkstoffgemisch mit antiinflammativer und spasmolytischer und Antistresswirkung bestehend aus einer Fraktion spezifischer Peptide mit einer relativen Molmasse bis zu 10.000 Dalton und/oder essentiellen und nichtessentiellen Aminosäuren wird aus den somatischen Zellen
- durch Inkubation bei den für die Ursprungsspezies typischen Temperaturen von 20 bis 39 °C über einen Zeitraum von 2 - 8 Tagen,
- anschließender Lyse,
- Ernte zusammen mit dem Erhaltungsmedium und
- Abtrennung von Bestandteilen, die eine Molmasse über 10.000 Dalton aufweisen, sowie geichzeitige Gewinnung der Fraktion spezifischer Peptide mit Molgewichten bis 10.000 Dalton und/oder der Fraktion mit essentiellen und nichtessentiellen Aminosäuren mittels Ultrazentrifugation bei einer Laufzeit von 24 Stunden.

Bevorzugt wird ein polyfunktionelles Wirkstoffgemisch einer Fraktion spezifischer Peptide mit einer relativen Molmasse zwischen 200 bis 6.000 Dalton eingesetzt. Dieses Gemisch wurde durch Aufkonzentration um den Faktor 1000 der gewonnenen Fraktion mit Proteinen < 10.000 Dalton mit physikalischen Mitteln erhalten. Die Inhaltsstoffe wurden mittels Massenspektroskopie analysiert.

Diese im Gemisch enthaltenen Peptide < 10.000 Dalton sind Peptide, die ebenfalls im menschlichen Organismus vorkommen, so dass damit ein "körpereigenes" Wirkstoffgemisch zur Verfügung steht, das gut verträglich ist. Körpereigene Peptide sind zahlreich bekannt und beschrieben. Im Sinne der vorliegenden Erfindung werden im Gemisch mit dem oben erwähnten polyfunktionellen Wirkstoffgemisch bevorzugt Peptide wirksam, die als homöostaseregulierend, stressregulierend, auf die glatte Muskulatur wirkend bzw. als Neuropeptide zu bezeichnen sind (Hecht, K., W-E Vogt, E. Wachtel, P.Oehme und M.G. Airapetjanz: Schlafregulierende Peptide. Beiträge zur Wirkstoffforschung. Heft 37 dito, 1990), (Oehme P., H. Bienert, K. Hecht, J. Bergmann: Substanz P. Beiträge zur Wirkstoffforschung. Heft 12 dito, 1981), (Marsan, C.A., W.Z. Trazyk (eds): Neuropeptides and Neural Transmission. International Brain Research Organosation (IBRO) Monograph Series: Volumen 7. Raven Press New York) Oehme , P.H. Löwe, E. Göres (Hrsg): Peptide und Adaptions-Grundlagenforschung und klinische Aspekte. Beiträge zur Wirkstoffforschung, Heft 36, Akademie-Industriekomplex Arzneimittelforschung Berlin (1990) Oehme, P.H. Löwe, E. Göres (Hrsg): Peptides in the Nervous System, Beiträge zur Wirkstoffforschung, Heft 24 (1985).

Mit diesem polyfunktionellen Wirkstoffgemisch wurde ein gesundheitsschützender Tabakrauchantagonist gefunden, der bevorzugt als Inhalat (Adhäsionsprinzip, Vernebelung) über einen Mundstückfilter der Rauchware (Zigarette, Zigarre, Zigarillo, Tabakpfeife u.a.) eingebracht und dem Körper zugeführt wird.

Das polyfunktionelle Wirkstoffgemisch wird dazu in das Mundstück (Filter) der Rauchwaren (Zigarette, Zigarre, Zigarillo, Tabakpfeife u.a.) im Produktionsprozess als zu vemebelndes Substrat eingebracht und während des Rauchens nach dem Adhäsionsprinzip vernebelt und auf diese Weise mit dem Tabakrauch vermischt in den Körper eingebracht.

Die gleichzeitige Anwendung mit der Rauchware führt zu keiner Geschmacksbeeinträchtigung für den Raucher. Darüber hinaus hat das polyfunktionelle Wirkstoffgemisch keine toxisch relevante Wirkung. Es ist bereits in sehr niedrigen Konzentrationen (10⁻⁶ bis 10⁻⁹ g/kg Körpergewicht) gesundheitsrelevant, quasi mittels bioaktiver Triggerfunktion, wirksam.
Das polyfunktionelle Wirkstoffgemisch hat spezifische Wirkeigenschaften, die als entzündungshemmend, durchblutungsfördernd, resorptions- permeabilitäts-stimulierend, spasmenlösend, immunmodulierend, disstressreduzierend, eustressstimulierend und homöostaseregulierend zu bezeichnen sind.
Die Dosis-Wirkung-Beziehung folgt vorzugsweise dem Prinzip einer Hyperbelkurve. Es wird nach erfolgter bioaktiver Triggerfunktionswirkung schnell im Körper abgebaut, so dass Überdosierungen auch bei starken Rauchern nicht möglich sind. Eine Resistenzentwicklung gegenüber pathogenen Erregern ist nicht nachgewiesen worden.

Es führt zur Abschwächung, Beseitigung und Verhinderung von Tabakrauchschadstoffinduzierten Entzündungen der Schleimhäute, von Regulationsstörungen der Organsysteme mit glatter Muskulatur (Blutgefäße, Bronchien, Ösophagus, Blase, Magen, Därme u.a.), der Funktionen des Herzmuskels, des zentralen und peripheren Nervensystems.

An Beispielen wird die Erfindung näher erläutert

### Ausführungsbeispiele

### 1. Herstellung des Gemisches

### Beispiel 1

Die diploide Kälbernieren-Zellinie MDBK (ATCC-No. CCL 22) wird entsprechend ihrer Vermehrungsrate 1 : 4 in DMEM mit 8 % Neugeborenen-Kälberserum verbracht und in Rollerflaschen bei 38 °C inkubiert. Größere Mengen werden nach Anpassung der Zellen günstigerweise in Spinnerkultur-gefäßen oder in Fermentoren angezüchtet. Nach etwa 3-4 Tagen ist die Kultur optimal ausgewachsen. Das Anzüchtungsmedium wird entfernt und durch MEM ohne Serum ersetzt.
Die Zellkulturen werden für weitere 5 - 7 Tage inkubiert. Mit zunehmender Inkubationszeit verarmt der intrazelluläre Pool an essentiellen Nährstoffen, gleichzeitig werden im MEM Mediatoren und niedermolekulare Stoffwechselendprodukte angereichert. Die Akkumulation von Stoffwechselprodukten und der intrazelluläre Mangel an essentiellen Nährstoffen stellt letztendlich für die alternde Zellkultur einen Stressfaktor da auf den sie mit durch die Synthese verschiedener "Stress-Proteine" reagiert. Diese werden in der Zelle angereichert. Die Zellkulturen werden während der Anreicherungsphase regelmäßig lichtmikroskopisch untersucht. Nach Ablauf der Anreicherungsphase dürfen keine zytopathischen Veränderungen sichtbar sein. Die Anreicherungsphase wird mit der Alkalisierung des MEM mit NaOH auf pH 10 und der Temperaturerhöhung auf 50 °C für 60 min beendet.
Statt der pH-Wert Verschiebung hat sich auch das Abfrieren der Zellen bewährt. Das geschieht günstigerweise bei -20 °C. Nach dieser Zeit können die abgestorbenen Zellen ohne Mühe von der Kulturflaschenoberfläche abgeschüttelt werden.
Temperaturerhöhung und pH-Shift haben keinen Einfluß auf die biologische Aktivität des Modulatorengemisches.
Nun wird das Erhaltungsmedium mit den extra- und intrazellulären Modulatoren durch Stufenfiltration (< 9 nm) von Zelldetritus und partikulären Bestandteilen befreit. Das gewonnene Filtrat enthält das Gemisch von Modulatoren, weitgehend befreit von Membranbestandteilen. Es kann steril ohne Aktivitätsverlust für mehrere Wochen bei 4 °C aufbewahrt werden bzw. eingefroren (- 20 °C und tiefer) für mindestens ein Jahr.

Durch Ultrafiltration mit handelüblichen Geräten, wobei die Porengröße der Filtrationsmembranen globuläre Moleküle abhalten, werden Fraktionen mit einer relativen Molmasse ab etwa 10.000 zurückgehalten. Im Retentat befinden sich alle extra- und intrazellulären Stoffe mit einer relativen Molmasse für globuläre Proteine von mehr als 10.000 und Partikel mit einen Durchmesser von weniger als 9 nm.

Das erfindungsgemäße polyfunktionelle Wirkstoffgemisch, bestehend aus der Fraktion spezifischer Peptide bis zu einem Molgewicht von 10.000 Dalton sowie aus essentiellen und nichtessentiellen Aminosäuren wurde mittels Ultrazentrifuge bei einer Laufzeit von 24 Stunden aus dem Proteingemisch gewonnen (analog DE 195 12 227 C1 vom 31.10.1996). Die Fraktionen mit Stoffen bis zu 10.000 Dalton Molgewicht wurden um den Faktor 1000 aufkonzentriert und anschließend mittels Massenspektroskopie auf ihre Inhaltsstoffe analysiert. Im erhaltenen Spektrum zeigte sich ein besonders hohes Niveau der Stoffe mit Molgewichten von 200 bis 6000 Dalton.

Das so angereicherte polyfunktionelle Wirkstoffgemisch kann steril ohne Aktivitätsverlust für mehrere Wochen bei 4 °C aufbewahrt werden bzw. eingefroren (- 20 °C und tiefer) für mindestens ein Jahr. Es kann auch in üblicher Weise lyophilisiert werden. Die Reinheit, Unschädlichkeit und Wirksamkeit des erfindungsgemäßen Modulatorengemisches wird durch entsprechende Kontrolluntersuchungen überprüft. Die Reinheits- und Unschädlichkeitsprüfungen erfolgen nach den vorgeschriebenen Methoden der Europäischen Pharmakopoe bzw. des DAB 10. Proteinbestimmung und Polyacrylgel-Elektrophorese werden nach altbekannten Standardprotokollen durchgeführt.

### Reinheit

1. Prüfung auf Sterilität gemäß Europäischer Pharmakopoe / DAB 10
2. Bestimmung des Proteingehaltes nach Lowry
3. Auftrennung und Quantifizierung der Hauptproteine mittels Polyacryamid-Gelelektrophorese unter denaturierenden Bedingungen.

### Unschädlichkeit

1. Prüfung auf Pyrogenfreiheit gemäß Europäischer Pharmakopoe / DAB 10
2. Prüfung auf akute Toxizität gemäß Europäischer Pharmakopoe / DAB 10
3. Prüfung auf Teratogenität gemäß Europäischer Pharmakopoe / DAB 10

Eine typische Präparation ist steril, pyrogenfrei, nicht toxisch und nicht teratogen. Es enthält etwa 2 - 3 mg Protein pro ml.

### Beispiel 2

Hühnerfibroblasten, gewonnen durch tryptische Zellvereinzelung von Hühnerembryonen, werden gemäß Beispiel 1 in Rollerflaschen kultiviert. Anreicherung, Ernte und Aufbereitung sowie Prüfung auf Identität, Unschädlichkeit und Wirksamkeit erfolgte gemäß Beispiel 1.
Es werden im wesentlichen die gleichen Ergebnisse wie in Beispiel 1 erzielt.

### Beispiel 3

Eine diploide, begrenzt wachsende Tubenepithel-Zellinie vom Schwein (Latzke,1993) wurde gemäß Beispiel 1 stationär kultiviert. Anreicherung, Ernte und Aufbereitung sowie Prüfung auf Identität, Unschädlichkeit und Wirksamkeit erfolgte gemäß Beispiel 1.

### 2. Anwendungsbeispiele

### Beispiel 4

In einer privaten allgemeinen ärztlichen Praxis für Alternativmedizin wurde 22 Patienten mit akuter Infektion der Atemwege das beschriebene polyfunktionelle Wirkstoffgemisch auf freiwilliger Basis als einmaliges Inhalat verabreicht. Innerhalb von 24 Stunden war die Symptomatik nicht mehr nachweisbar. Dagegen dauerte bei den Patienten (n=28), die zur gleichen Zeit die gleiche Symptomatik auswiesen und mit herkömmlichen therapeutischen Mitteln behandelt wurden, dieser Krankheitsprozess 3-7 Tage.

### Beispiel 5 - Einzelfälle

5,1 Bronchopneumonie: Eine 48 jährige Patientin, bei der seit über einer Woche eine Bronchitis und eine nicht erkannte Bronchopneumonie bestanden, wurde auf freiwilliger Basis täglich 2x das beschriebene multifunktionelle Wirkstoffgemisch als Inhalat verabreicht. Bereits nach 2 Inhalationen ließen die Beschwerden nach. Innerhalb einer Woche war die Symptomatik weitergehend abgeklungen.
5,2 Virusbronchitis: 76 jähriger Patient litt ca. 2 Wochen an einer therapieresistenten chronischen Virusbronchitis und starkem nächtlichen Reizhustenanfällen. Nach der ersten Inhalation des beschriebenen polyfunktionellen n Wirkstoffgemisches war der nächtliche Reizhusten gemindert, nach zwei Inhalationen war die gesamte Bronchitis beseitigt.
5,3 76 jähriger Patient litt seit Jahren an häufiger auftretenden Nasenblutungen (offensichtlich allergischen Charakters). Nach acht täglich 2x erfolgten Inhalationen des beschriebenen polyfunktionellen n Wirkstoffgemisches trat das Nasenbluten nicht mehr auf. Die Nachkontrollzeit beträgt derzeitig 6 Monate.

### Beispiel 6 - Hyperfunktion, Rauchen, Wirkstoffinhalation

An 12 Freiwilligen wurde während des Rauchens einer Zigarette mittels eines sehr sensiblen Herzfunktionswarnsystems auf EKG-Basis (Jumatow Moskau) die Wirkung des beschriebenen polyfunktionellen Wirkstoffgemisches überprüft. Einmal wurde die Zigarette ohne und einmal mit dem polyfunktionellen Wirkstoffgemisch geraucht. Während beim Rauchen ohne Wirkstoffinhalat das Herzfunktionswarnsystem das Risiko signalisierte, blieb diese Warnung bei gleichzeitiger Inhalation des Zigarettenrauches und des vernebelten Wirkstoffes aus,

## Patentansprüche

1. Verwendung eines polyfunktionellen Wirkstoffgemisches mit antiinflammatorischer, spasmolytischer und Antistresswirkung, das sich zusammensetzt aus einer Fraktion spezifischer Peptide mit Molgewichten bis 10 000 Dalton und einer Fraktion mit essentiellen und nichtessentiellen Aminosäuren, und welches aus einem multifaktoriellen Abwehr-Modulatorengemisch gewonnen wurde, erzeugt durch
- Inkubation bei den für die Ursprungsspezies typischen Umgebungstemperaturen von 20 bis 39 °C über einen Zeitraum von 2 - 8 Tagen,
- anschließender Lyse,
- Ernte zusammen mit dem Erhaltungsmedium und
- Abtrennung von Zellbestandteilen mittels Ultrazentrifugation bei einer Laufzeit von 24 Stunden, die eine Molmasse über 10 000 Dalton aufweisen, und
- Gewinnung der Fraktion spezifischer Peptide mit Molgewichten bis 10 000 Dalton und der Fraktion mit essentiellen und nicht essentiellen Aminosäuren,
zur Herstellung von Tabakrauchschadstoffantagonisten.

2. Verwendung nach Anspruch 1, **dadurch gekennzeichnet, dass** eine Fraktion spezifischer Peptide mit Molgewichten zwischen 200 bis 6000 Dalton und/oder eine Fraktion mit essentiellen und nicht essentiellen Aminosäuren eingesetzt werden.

3. Verwendung nach Anspruch 1 **dadurch gekennzeichnet, dass** Gemische von homöostaseregulierenden, auf die glatte Muskulatur wirkenden, stressregulierenden Peptiden sowie von Neuropeptiden enthalten sind und verwendet werden.

4. Verwendung nach einem der Ansprüche 1 bis 3 , **dadurch gekennzeichnet, dass** das Gemisch in niedrigen Konzentrationen, vorzugsweise in Dosierungen von 10⁻⁶ bis 10⁻⁹ g/kg Körpergewicht, eingesetzt wird und seine Wirkung vor allem in Form einer bioaktiven Triggerfunktion erfolgt.

5. Verwendung nach einem der Ansprüche 1 bis 4, **dadurch gekennzeichnet, dass** die Dosis-Wirkungsbeziehung dem Prinzip einer Hyperbelkurve folgt, wodurch eine Überdosierung ausgeschlossen ist.

6. Verwendung nach einem der Ansprüche 1 bis 5, **dadurch gekennzeichnet, dass** das Gemisch in einem spezifischen Filter (spezifisches Mundstück) der Rauchwaren (Zigarette, Zigarre, Zigarillo, Tabakpfeife u.a.) verwendet wird, und bei der Herstellung der Rauchware in diese eingebracht, wobei es beim Rauchen inhaliert wird.

7. Verwendung nach einem der Ansprüche 1 bis 6 **dadurch gekennzeichnet, dass** er selbst nicht gesundheits- und suchtgefährdend ist, sowie den Rauchgenuß nicht verändert.

8. Verwendung nach einem der Ansprüche 1 bis 7, **dadurch gekennzeichnet, dass** das polyfunktionelle Wirkstoffgemisch zur Abschwächung, Beseitigung und Verhinderung Tabakrauchschadstoff-induzierter
- Entzündungen der Schleimhäute
- Regulationsstörungen
- der Funktion der Organsysteme mit glatter Muskulatur (Bronchien, Blutgefäße, Ösophagus, Blase, Magen, Därme u.a.)
- der Funktionen der Herztätigkeiten sowie
- der Funktionen des zentralen und peripheren Nervensystems verwendet wird.

9. Spezifische Filter für Rauchwaren, **dadurch gekennzeichnet, dass** sie ein polyfunktionelles Wirkstoffgemisch nach einem der Ansprüche 1 bis 4 mit gesundheitsschützender Funktion beim Tabakrauchen aufweisen.

## Claims

1. Use of a polyfunctional active substance mixture with anti-inflammatory, spasmolytic and anti-stress action comprised of a fraction of specific peptides with molar weights of up to 10,000 dalton and of a fraction of essential and non-essential amino acids, and said polyfunctional active substance mixture was obtained from a multifactorial immune-modulator mixture: by way of
- incubation at the temperatures that are typical for the original species of 20 to 39 degree Celsius for a time period of 2 to 8 days;
- followed by lysis;
- harvested together with the conservation medium; and,
- separation of cell components which have a molar mass of over 10,000 dalton by way of high-speed centrifuging at a running time of 24 hours; and,
- gaining the fraction of specific peptides with molar weights of up to 10,000 dalton and/or of the fraction of essential and non-essential amino acids
as an antagonist against the harmful substances contained in tobacco smoke.

2. Use as claimed in claim 1 wherein a fraction of specific peptides with molar weights of between 200 and 6,000 dalton and/or a fraction of essential and non-essential amino acids are applied.

3. Use as claimed in claim 1 wherein mixtures of peptides, as well as neuropeptides contained therein, are applied that regulate homeostasis, act upon the smooth muscles and regulate stress.

4. Use as claimed in one of the claims 1 to 3 wherein the mixture is applied at low concentrations, preferably at a dosage of 10⁻⁶ to 10⁻⁹ g/kg of body weight and wherein its action occurs primarily in the form of a bioactive triggering function.

5. Use as claimed in one of the claims 1 to 4 wherein the dose-action relationship follows the principle of a hyperbolic curve whereby an overdose is impossible.

6. Use as claimed in one of the claims 1 to 5 wherein the mixture is applied in a specific filter (specific mouth tip) of the tobacco product (cigarette, cigar, cigarillo, tobacco pipe, etc.) and wherein the mixture is incorporated into the tobacco product at the time of the manufacture of the tobacco product and inhaled during smoking.

7. Use as claimed in one of the claims 1 to 6 wherein it itself is not harmful to the smoker's health, nor does it constitute a risk for addiction, and it does not change the smoking enjoyment.

8. Use as claimed in one of the claims 1 to 7 wherein the polyfunctional active substance mixture is applied for ameliorating, eliminating and preventing:
- inflammations of the mucous membranes;
- regulatory disorders
■ of the function of the organ systems with smooth muscles (bronchial system, blood vessels, esophagus, bladder, stomach, intestines, etc.);
■ of the myocardial functions; and, -
■ of the functions of the central and peripheral nerve system
induced by the harmful substances contained in tobacco smoke.

9. Specific filters for tobacco products wherein these filters comprise a polyfunctional active substance mixture as claimed in one of the claims 1 to 4 featuring a health-protecting function during the smoking of tobacco.

## Revendications

1. Emploi d'un mélange de principes actifs polyfonctionnel à effet anti-inflammatoire, spasmolytique et antistress pour la fabrication d'antagonistes aux toxines de la fumée de tabac, qui se compose d'une fraction de peptides spécifiques aux poids molaires allant jusqu'à 10.000 daltons et d'une fraction contenant des acides aminés essentiels et non-essentiels, et qui a été extrait d'un mélange de modulateurs de défense multifactoriel, fabriqué par
- incubation aux températures ambiantes typiques pour l'espèce originale de 10 à 39 °C durant 2 à 8 jours,
- suivie d'une lyse,
- récolte en même temps que le milieu de conservation et
- séparation d'éléments cellulaires au moyen de l'ultracentrifugation réalisée durant 24 heures, éléments qui ont une masse molaire de plus de 10.000 daltons, et
- extraction de la fraction de peptides spécifiques aux poids molaires allant jusqu'à 10.000 daltons et de la fraction contenant des acides aminés essentiels et non essentiels.

2. Emploi selon la revendication 1, **se caractérisant par le fait qu'**une fraction de peptides spécifiques aux poids molaires entre 200 et 6.000 daltons et/ou une fraction contenant des acides aminés essentiels et non essentiels sont utilisées.

3. Emploi selon la revendication 1 **se caractérisant par le fait que** des mélanges de peptides régulateurs du stress, de l'homéostasie, agissant sur la musculature lisse ainsi que de neuropeptides sont contenus et utilisés.

4. Emploi selon l'une des revendications 1 à 3, **se caractérisant par le fait que** le mélange est utilisé en faibles concentrations, de préférence dans des dosages de 10⁻⁶ à 10⁻⁹ g/kg de poids du corps et que son effet se fera surtout sous forme d'une fonction trigger bioactive.

5. Emploi selon l'une des revendications 1 à 4, **se caractérisant par le fait que** le rapport du dosage et de l'effet suit le principe d'une courbe hyperbole, ce qui exclut un surdosage.

6. Emploi selon l'une des revendications 1 à 5, **se caractérisant par le fait que** le mélange est utilisé dans un filtre spécifique (embout spécifique) des articles contenant du tabac (cigarette, cigare, cigarillo, pipe et autres) et que ce mélange est intégré dans ces articles lors de leur fabrication, pour être inhalé pendant que la personne fume.

7. Emploi selon l'une des revendications 1 à 6, **se caractérisant par le fait que** le mélange n'est lui-même pas toxique ni ne crée un état de dépendance, et ne modifie pas la consommation du tabac.

8. Emploi selon l'une des revendications 1 à 7, **se caractérisant par le fait que** le mélange de principes actifs polyfonctionnel est utilisé pour réduire, éliminer et empêcher les affections induites par les toxines de la fumée du tabac suivantes :
- les inflammations des muqueuses
- les troubles de régulation
■ des fonctions des systèmes d'organes à musculature lisse (bronches, vaisseaux sanguins, oesophage, vessie, estomac, intestins et autres)
■ des fonctions cardiaques ainsi que
■ des fonctions du système nerveux central et périphérique.

9. Filtre spécifique pour les articles contenant du tabac, **se caractérisant par le fait qu'**ils contiennent un mélange de principes actifs polyfonctionnel selon l'une des revendications 1 à 4 dont la fonction est la protection de la santé pendant que la personne fume du tabac.
